# EUROPEAN PATENT SPECIFICATION

(11) **EP 3 889 131 B1**
(45) Date of publication and mention of the grant of the patent: **25.06.2025**
(21) Application number: 19890332.0
(22) Date of filing: 05.11.2019
(51) Int. Cl.: C07C 231/12, C07C 231/24, C07C 233/05

(54) **METHOD FOR PRODUCING N-VINYLCARBOXYLIC AMIDE**
VERFAHREN ZUR HERSTELLUNG VON N-VINYLCARBONAMID
PROCÉDÉ DE PRODUCTION D'AMIDE N-VINYLCARBOXYLIQUE

(30) Priority: 29.11.2018 JP 2018224143
(43) Date of publication of application: 06.10.2021
(73) Proprietor: Resonac Corporation, Tokyo (JP)
(72) Inventor: TANAKA, Naoyuki, Tokyo 105-8518 (JP); KAKO, Toshihiro, Tokyo 105-8518 (JP); KOBAYASHI, Takamitsu, Tokyo 105-8518 (JP)
(74) Representative: Strehl & Partner mbB
(86) International application number: PCT/JP2019/043185
(87) International publication number: WO 2020/110614

(56) References cited:
- EP-A1- 2 386 538
- WO-A1-2007/018279
- WO-A1-2017/002494
- WO-A1-2018/041615
- JP-A- 2002 167 369
- JP-A- H03 181 452
- JP-A- H09 323 964
- JP-A- S61 106 546

## Description

### Technical Field

The present invention relates to a method for producing N-vinylcarboxylic acid amide in which N-(1-alkoxyethyl)carboxylic acid amide is used as a raw material.

### Background Art

In relation to methods for producing N-vinylcarboxylic acid amide, a large number of methods have been proposed (see PTL 1, for example). In a production method described in PTL 1, a method for producing N-vinylcarboxylic acid amide by evaporating and thermally decomposing a raw material of N-(2-alkoxyethyl)carboxylic acid amide is disclosed. Thus, in the method for producing N-vinylcarboxylic acid amide by the thermal decomposition, for example, for main causes such as change in pressure due to a mechanical trouble and stop of supply from a heat source, the raw material is liquefied in an evaporator or a piping connecting the evaporator and a thermal decomposition reactor, and the liquefied raw material flows into the thermal decomposition reactor at a high temperature. In this case, tar or solid aggregates (referred to also as coagulum) might be generated. The generation of coagulum results in a problem of the thermal decomposition reactor being blocked, thereby making it difficult to perform a stable operation. EP 2 386 538 A1, WO 2007/018279 A1, and WO 2018/041615 A1 disclose a method for producing N-vinylcarboxylic acid amide, comprising a feeding step of feeding N-(1-alkoxyethyl)carboxylic acid amide as a raw material to an evaporator, an evaporation step of evaporating the raw material to form a vaporized raw material, and a thermal decomposition step of feeding the vaporized raw material to a thermal decomposition reactor to thermally decompose the raw material.

### Citation List

### Patent Literature

PTL 1: WO 2017/002494 (A1)

### Summary of Invention

### Technical Problem

An object of the present invention is to solve the above problems and provide a method for producing N-vinylcarboxylic acid amide, in which a problem of a thermal decomposition reactor being blocked with coagulum can be inhibited, and an operation can be stably and continuously performed for a long period of time.

### Solution to Problem

The present invention is defined in the appended claims.

### Advantageous Effects of Invention

According to the present invention, a method for producing N-vinylcarboxylic acid amide can be provided, in which a problem of a thermal decomposition reactor being blocked with coagulum can be inhibited, and an operation can be stably and continuously performed for a long period of time.

### Brief Description of Drawings

[Fig. 1] Fig. 1 is a flowchart showing a method for producing N-vinylcarboxylic acid amide of the present invention.
[Fig. 2] Fig. 2 is a schematic overall view in a case where an evaporator is a falling film evaporator, in the method for producing N-vinylcarboxylic acid amide according to the present invention.
[Fig. 3] Fig. 3 is a schematic overall view in a case where the evaporator is a forced film evaporator, in the method for producing N-vinylcarboxylic acid amide according to the present invention.

### Description of Embodiments

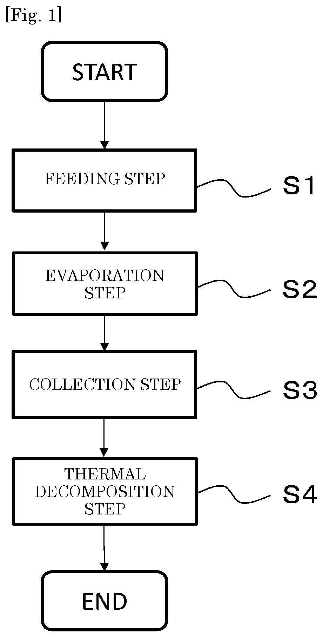

As shown in Fig. 1, a method for producing N-vinylcarboxylic acid amide according to an embodiment of the present invention comprises a feeding step S1, an evaporation step S2, a collection step S3, and a thermal decomposition step S4. Hereinafter, the method for producing N-vinylcarboxylic acid amide according to the embodiment of the present invention will be described in detail.

### (Feeding Step S1)

The feeding step S1 is a step of feeding N-(1-alkoxyethyl)carboxylic acid amide as a raw material to an evaporator.

In the method for producing N-vinylcarboxylic acid amide according to the embodiment of the present invention, as the raw material, preferably N-(1-alkoxyethyl)carboxylic acid amide represented by the following general formula (I) is used. 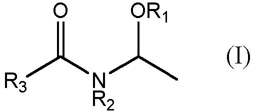 wherein R₁ represents a C₁ to C₅ alkyl group, R₂ represents a hydrogen atom or C₁ to C₅ alkyl group, and R₃ represents a C₁ to C₅ alkyl group.

Examples of N-(1-alkoxyethyl)carboxylic acid amide include N-(1-methoxyethyl)acetamide, N-(1-methoxyethyl)-N-methylacetamide, N-(1-ethoxyethyl)acetamide, N-(1-ethoxyethyl)-N-methylacetamide, N-(1-propoxyethyl acetamide, N-(1-isopropoxyethyl)acetamide, N-(1-butoxyethyl)acetamide, N-(1-isobutoxyethyl)acetamide, N-(1-methoxyethyl)propionamide, N-(1-ethoxyethyl)propionamide, N-(1-propoxyethyl)propionamide, N-(1-isopropoxyethyl)propionamide, N-(1-butoxyethyl)propionamide, N-(1-isobutoxyethyl)propionamide, N-(1-methoxyethyl)isobutyl amide, N-(1-ethoxyethyl)isobutyl amide, N-(1-propoxyethyl)isobutyl amide, N-(1-isopropoxyethyl)isobutyl amide, N-(1-butoxyethyl)isobutyl amide, and N-(1-isobutoxyethyl)isobutyl amide. The examples preferably include N-(1-methoxyethyl)acetamide, N-(1-isopropoxyethyl)acetamide and N-(1-methoxyethyl)isobutyl amide, and more preferably include N-(1-methoxyethyl)acetamide.

In the feeding step S1, a feeding speed of the raw material to the evaporator, which depends on a size and capacity of the evaporator, is preferably from 5 to 350 kg/h, more preferably from 10 to 150 kg/h, and further preferably from 15 to 80 kg/h from a viewpoint of stably evaporating the raw material.

### (Evaporation Step S2)

The evaporation step S2 is a step of evaporating, by the evaporator, the raw material, to form a vaporized raw material.

There are not any special restrictions on the evaporator for use in the evaporation step S2, but it is preferable from a viewpoint of efficiently evaporating the raw material that the evaporator is a falling film evaporator or a forced film evaporator.

The evaporation step S2 can be performed under reduced pressure or under normal pressure and is preferably performed under reduced pressure. Specifically, the evaporation step S2 is performed with a reaction pressure preferably from 1 to 30 kPa, more preferably from 3 to 25 kPa, and further preferably from 5 to 22 kPa.

The evaporation step S2 is required to be performed specifically at a temperature to heat and evaporate the raw material and form the vaporized raw material, preferably from 100 to 200°C, more preferably from 110 to 190°C, and further preferably from 120 to 180°C.

### (Collection Step S3)

The collection step S3, between the evaporation step S2 and the thermal decomposition step S4, is a step of collecting, from the raw material fed to the evaporator, a liquid raw material that is not vaporized and a liquid raw material comprising a part of the vaporized raw material that is liquefied. The vaporized raw material in a vaporized state is for use as it is in the thermal decomposition step.

If even a small amount of liquid raw material is introduced into a thermal decomposition reactor, coagulum is generated. The thermal decomposition reactor is blocked with coagulum, and an operation cannot be continued. Even when a total volume of the thermal decomposition reactor is not blocked with coagulum, the operation cannot be continued if a small volume of the thermal decomposition reactor on a raw material introduction side is blocked. In the collection step S3, the liquid raw material is collected in a collection pot outside a system of a production apparatus, which can prevent the liquid raw material from being introduced into the thermal decomposition reactor. As a result, it is possible to continue the operation for a long period of time.

Examples of main causes for the generation of the liquid raw material during the operation of the production apparatus include fluctuations in operating pressure due to mechanical failure and operation mistake, stop or lack of heat source of the evaporator due to the mechanical failure and operation mistake, poor evaporation due to a heat transfer area decreased by scale (black skin) generated in the evaporator, and introduction of the raw material during insufficient heating of the apparatus at start of the operation.

### (Thermal Decomposition Step S4)

The thermal decomposition step S4 is a step of feeding the vaporized raw material to the thermal decomposition reactor, to thermally decompose the raw material. There are not any special restrictions on flow of the vaporized raw material to be fed to the thermal decomposition reactor as long as the material flows into the thermal decomposition reactor, and the flow may be ascending flow or descending flow.

The thermal decomposition step S4 can be performed under reduced pressure or under normal pressure and is preferably performed under reduced pressure. Specifically, the thermal decomposition step S4 is performed with a reaction pressure preferably from 1 to 30 kPa, more preferably from 3 to 25 kPa, and further preferably from 5 to 20 kPa.

From a viewpoint of efficiently performing thermal decomposition, the thermal decomposition step S4 is performed at a temperature preferably from 250 to 500°C, more preferably from 300 to 480°C, and further preferably from 350 to 450°C.

From a viewpoint of securely performing the thermal decomposition, a staying time in the thermal decomposition step S4 is preferably from 0.1 to 10 seconds, more preferably from 0.2 to 9 seconds, and further preferably from 0.3 to 8 seconds.

It is preferable from the viewpoint of efficiently performing the thermal decomposition that the thermal decomposition reactor includes a multi-tube structure.

The raw material to be fed to the thermal decomposition reactor is the vaporized raw material subjected to the collection step. The vaporized raw material subjected to the collection step is fed to the thermal decomposition reactor, so that the liquid raw material can be prevented from being introduced into the thermal decomposition reactor, and the generation of coagulum can be inhibited.

Preferably, N-vinylcarboxylic acid amide obtained by the above steps is represented by the following general formula (II) and corresponds to N-(1-alkoxyethyl)carboxylic acid amide that is a suitable raw material represented by the general formula (I). 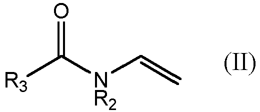 wherein R₂ represents a hydrogen atom or C₁ to C₅ alkyl group, and R₃ represents a C₁ to C₅ alkyl group.

Examples of N-vinylcarboxylic acid amide include N-vinylacetamide, N-methyl-N-vinylacetamide, N-vinylpropionamide, N-methyl-N-vinylpropionamide, N-vinylisobutylamide and N-methyl-N-vinylisobutylamide, and preferably include N-vinylacetamide.

### (First Production Apparatus)

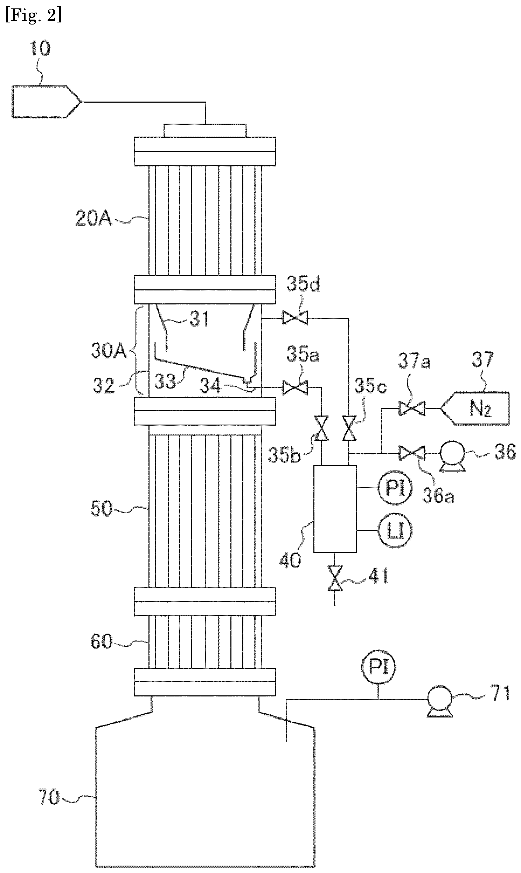

As shown in Fig. 2, a first production apparatus that performs the method for producing N-vinylcarboxylic acid amide according to the embodiment of the present invention comprises a raw material feeding device 10 that performs the feeding step S1, an evaporator (a falling film evaporator) 20A that performs the evaporation step S2, a raw material collection device 30A that performs the collection step S3, a thermal decomposition reactor 50 that performs the thermal decomposition step S4, a cooler 60 that cools and liquefies a reactant thermally decomposed in the thermal decomposition step S4, and a reaction solution receiver 70 that stores the reactant liquefied in the cooler 60. The falling film evaporator is an evaporator that evaporates the liquid raw material flowing like a film downward along an inner surface of each evaporation tube.

In the raw material collection device 30A, a collection pot 40 that stores the collected liquid raw material is disposed.

In the reaction solution receiver 70, a pressure pump 71 is provided, and a pressure of the whole production apparatus can be adjusted with the pressure pump 71. The pressure of the whole production apparatus can be confirmed with a pressure indicator PI provided in the reaction solution receiver 70.

The raw material collection device 30A may include a structure that inhibits the liquid raw material from being introduced into the thermal decomposition reactor.

For example, as shown in Fig. 2, it is preferable that the raw material collection device 30A is configured to comprise a cylindrical part 32 through which the vaporized raw material is distributed, a collecting section 33 with which flow of the vaporized raw material flowing through the cylindrical part 32 partially or entirely collides, and that collects, from the raw material fed to the evaporator, the liquid raw material that is not vaporized and the liquid raw material comprising a part of the vaporized raw material that is liquefied, and a discharge piping 34 through which the liquid raw material collected in the collecting section 33 is discharged to outside the cylindrical part 32. It is preferable that the raw material collection device 30A further comprises a distribution inhibiting section 31 configured so that the vaporized raw material flowing through the cylindrical part 32 entirely collides with the collecting section 33.

It is preferable that the distribution inhibiting section 31 has a tapered shape that narrows down flow of the vaporized raw material toward downstream.

It is preferable that the collecting section 33 has a dish shape that receives the flow of the vaporized raw material, and has the shape including an accumulating portion that accumulates the liquid raw material.

According to the above configuration of the raw material collection device 30A, from the raw material fed to the evaporator, the liquid raw material that is not vaporized and the liquid raw material comprising the part of the vaporized raw material that is liquefied collide with the collecting section 33 and are collected, and the liquid raw materials are discharged through the discharge piping 34 to outside the cylindrical part 32. The vaporized raw material that collides with the collecting section 33 passes through a space between the collecting section 33 and the distribution inhibiting section 31 toward the thermal decomposition reactor. That is, according to the above configuration of the raw material collection device 30A, the vaporized raw material that collides with the collecting section 33 and from which the liquid raw material is collected is introduced into the thermal decomposition reactor.

The liquid raw material collected in the raw material collection device 30A is sent to the collection pot 40. The collection pot 40 can control the liquid raw material with gate valves 35a and 35b. Furthermore, the collection pot 40 can adjust a pressure of the collection pot 40 through a pressure pump 36 and the gate valves 35a to 35d. The pressure of the collection pot 40 can be confirmed with a pressure indicator PI.

The liquid raw material collected in the raw material collection device 30A always flows through the gate valves 35a and 35b into the collection pot 40. An amount of the material to be collected into the collection pot 40 can be confirmed with a liquid level indicator LI such as a liquid level confirmation window installed in the collection pot 40. When collecting the liquid raw material stored in the collection pot 40, the gate valves 35b and 35c are closed, a nitrogen valve 37a is then opened to introduce nitrogen from a nitrogen supply device 37 into the collection pot 40 and to return to normal pressure, and an extraction valve 41 is opened. After the collection, the extraction valve 41 and the nitrogen valve 37a are closed, a gate valve 36a is opened to adjust a pressure to the same pressure as in the whole production apparatus with the pressure pump 36, the gate valve 35c is then opened, and the gate valve 35b is next opened, to obtain a state where the liquid raw material can be collected.

A temperature of the raw material collection device 30A is suitably selected from a temperature range in which the vaporized raw material generated in the evaporator 20A does not condense. A temperature of the collection pot 40 may only be a temperature at which the collected liquid raw material does not solidify.

It is preferable to feed, to the evaporator 20A, the liquid raw material collected in the raw material collection device 30A. In the first production apparatus shown in Fig. 2, the liquid raw material collected in the collection pot 40 is fed to the evaporator 20A. The collected liquid raw material is fed to the evaporator again, so that the raw material can be efficiently used.

As shown in Fig. 2, a configuration is preferable where a downstream end of the evaporator 20A is joined to an upstream end of the raw material collection device 30A. According to the configuration where the downstream end of the evaporator 20A is joined to the upstream end of the raw material collection device 30A, the vaporized raw material heated and evaporated in the evaporator 20A can be directly sent to the raw material collection device 30A. That is, a part of the vaporized raw material can be prevented from being liquefied between the evaporator 20A and the raw material collection device 30A.

As shown in Fig. 2, a configuration is preferable where a downstream end of the raw material collection device 30A is joined to an upstream end of the thermal decomposition reactor 50. According to the configuration where the downstream end of the raw material collection device 30A is joined to the upstream end of the thermal decomposition reactor 50, the vaporized raw material from which the liquid raw material is collected in the raw material collection device 30A can be directly sent to the thermal decomposition reactor 50. That is, a part of the vaporized raw material can be prevented from being liquefied between the raw material collection device 30A and the thermal decomposition reactor 50.

### (Second Production Apparatus)

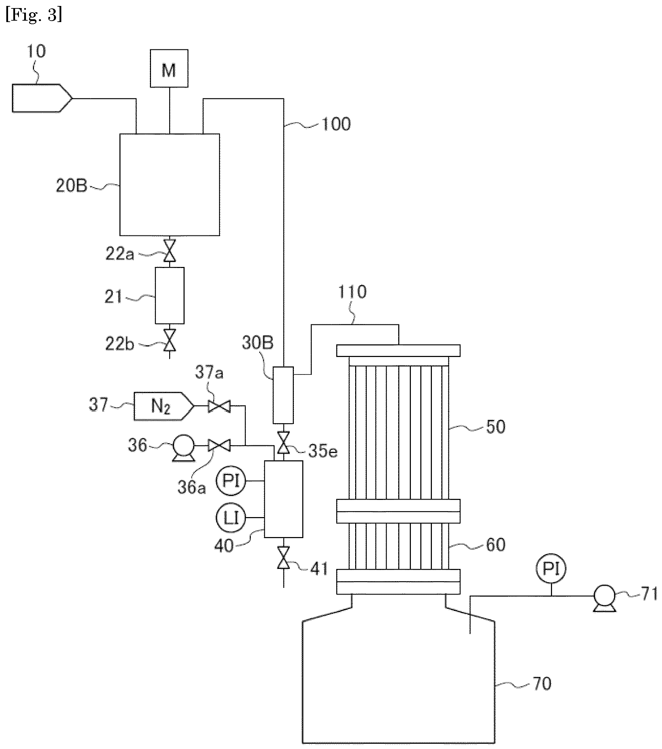

As shown in Fig. 3, a second production apparatus that performs the method for producing N-vinylcarboxylic acid amide according to the embodiment of the present invention comprises a raw material feeding device 10 that performs the feeding step S1, an evaporator (a forced film evaporator) 20B that performs the evaporation step S2, a raw material collection device 30B that performs the collection step S3, a thermal decomposition reactor 50 that performs the thermal decomposition step S4, a cooler 60 that cools and liquefies a reactant thermally decomposed in the thermal decomposition step S4, and a reaction solution receiver 70 that stores the reactant liquefied in the cooler 60. The forced film evaporator is an evaporator in which the liquid raw material flows inside like a film along an inner surface of each evaporation tube, a fan or the like is rotated with a motor to generate a propulsive force in the evaporation tube due to flow of air, and the film-like raw material in the evaporation tube flows forward forcibly with the propulsive force to be evaporated.

In the raw material collection device 30B, a collection pot 40 that stores the collected liquid raw material is disposed.

In the reaction solution receiver 70, a pressure pump 71 is provided, and a pressure of the whole production apparatus can be adjusted with the pressure pump 71. The pressure of the whole production apparatus can be confirmed with a pressure indicator PI provided in the reaction solution receiver 70.

It is preferable that in the evaporator 20B, a collection pot 21 is disposed to collect, from a raw material fed to the evaporator 20B, a liquid raw material that is not vaporized and a liquid raw material comprising a part of a vaporized raw material that is liquefied. From a viewpoint of facilitating the collection of the liquid raw material, it is preferable to dispose the collection pot 21 at a position lower than a position of the evaporator 20B. The collection pot 21 comprises a gate valve 22a that separates the evaporator 20B from the collection pot 21, and an extraction valve 22b that extracts the liquid raw material collected in the collection pot 21 to outside a system. The collection pot 21 can control the liquid raw material with the gate valve 22a and the extraction valve 22b. When collecting the liquid raw material that is not vaporized from the raw material fed to the evaporator 20B, the extraction valve 22b is closed, and the gate valve 22a is opened. When extracting the liquid raw material collected in the collection pot 21, the gate valve 22a is closed, and the extraction valve 22b is opened.

As shown in Fig. 3, a configuration is preferable where a downstream end of the evaporator 20B is connected to an upstream end of the raw material collection device 30B via a first piping 100, and a downstream end of the first piping 100 is at the lowest position in the first piping 100. According to the configuration where the downstream end of the first piping 100 is at the lowest position in the first piping 100, the liquid raw material that is not vaporized and the liquid raw material comprising a part of the vaporized raw material that is liquefied can be efficiently collected from the raw material fed to the evaporator and flowing through the first piping 100, in the raw material collection device 30B.

The liquid raw material collected in the raw material collection device 30B is sent to the collection pot 40. From the viewpoint of facilitating the collection of the liquid raw material, it is preferable to dispose the collection pot 40 at a position lower than a position of the raw material collection device 30B. The collection pot 40 can control the liquid raw material with a gate valve 35e and an extraction valve 41. Furthermore, in the collection pot 40, a pressure of the collection pot 40 can be adjusted with a pressure pump 36, the gate valve 35e and the extraction valve 41. The pressure of the collection pot 40 can be confirmed with a pressure indicator PI.

The liquid raw material collected in the raw material collection device 30B always flows through the gate valve 35e into the collection pot 40. An amount of the raw material to be collected in the collection pot 40 can be confirmed with a liquid level indicator LI such as a liquid level confirmation window installed in the collection pot 40. When collecting the liquid raw material stored in the collection pot 40, the gate valve 35e is closed, a nitrogen valve 37a is then opened to introduce nitrogen from a nitrogen supply device 37 into the collection pot 40 and to return to normal pressure, and the extraction valve 41 is opened. After the collection, the extraction valve 41 and the nitrogen valve 37a are closed, a gate valve 36a is opened to adjust a pressure to the same pressure as in the whole production apparatus with the pressure pump 36, and the gate valve 35e is then opened to obtain a state where the liquid raw material can be collected.

A temperature of the raw material collection device 30B is suitably selected from a temperature range in which the vaporized raw material generated in the evaporator 20B does not condense. A temperature of the collection pot 40 may only be a temperature at which the collected liquid raw material does not solidify.

It is preferable to feed, to the evaporator 20B, the liquid raw material collected in the raw material collection device 30B. In the second production apparatus shown in Fig. 3, the liquid raw material collected in the collection pot 40 is fed to the evaporator 20B. Furthermore, in the second production apparatus shown in Fig. 3, it is also preferable to feed, to the evaporator 20B, the liquid raw material collected in the collection pot 21. The collected liquid raw material is fed to the evaporator again, so that the raw material can be efficiently used.

In the production apparatus shown in Fig. 3, a configuration is preferable where a downstream end of the raw material collection device 30B is connected to an upstream end of the thermal decomposition reactor 50 via a second piping 110, and an upstream end of the second piping 110 is at the lowest position in the second piping 110. According to the configuration where the upstream end of the second piping 110 is at the lowest position in the second piping 110, even if a part of the vaporized raw material flowing through the second piping 110 is liquefied, the liquid raw material obtained by liquefying the part can be efficiently collected in the raw material collection device 30B.

In the production apparatus shown in Fig. 3, it is preferable that the second piping 110 is configured to be connected to a side surface or an upper surface of the raw material collection device 30B. According to the configuration where the second piping 110 is connected to the side surface or the upper surface of the raw material collection device 30B, the liquid raw material collected in the raw material collection device 30B can be inhibited from flowing into the second piping 110.

Also, in the second production apparatus shown in Fig. 3, similarly to the first production apparatus, it is preferable that the downstream end of the raw material collection device 30B is configured to be joined to the upstream end of the thermal decomposition reactor 50. That is, in the production apparatus shown in Fig. 3, it is preferable that, in place of the raw material collection device 30B, the gate valve 35e, the pressure pump 36, the collection pot 40, the extraction valve 41 and the second piping 110, the raw material collection device 30A, the gate valves 35a to 35d, the pressure pump 36, the collection pot 40 and the extraction valve 41 in Fig. 2 are provided. According to the configuration where the downstream end of the raw material collection device 30B is joined to the upstream end of the thermal decomposition reactor 50, the vaporized raw material from which the liquid raw material is collected in the raw material collection device 30B can be directly sent to the thermal decomposition reactor 50. That is, a part of the vaporized raw material can be prevented from being liquefied between the raw material collection device 30B and the thermal decomposition reactor 50.

### Examples

Hereinafter, the present invention will be further specifically described by way of examples.

### Example 1

A production apparatus shown in Fig. 2 was used.

As an evaporator, a falling film evaporator was used in which a large number of tubes were installed in an interior of a shell. Such a shell and tube evaporator comprising a structure where liquid flowed downward along inner walls of these tubes was used (a tube diameter: 25.4 mm, a tube length: 2500 mm, and a number of tubes: 22).

In the evaporator on a shell side, saturated vapor at 0.7 MPaG (a temperature of 170°C) was introduced, and on a tube side, N-(1-methoxyethyl)acetamide that was a liquid raw material was set to decompression conditions at 20 kPa with decompression equipment (a pressure pump) installed in a reaction solution receiver, fed to flow downward along the inner walls of the tubes at a feeding speed of 20 kg/h, and evaporated.

Flanges each having an outer diameter of 267.4 mm were installed to upper and lower parts of a raw material collection device and joined to the evaporator and a thermal decomposition reactor, respectively. The saturated vapor at 0.7 MPaG was introduced into a cylindrical part (a jacket) of the raw material collection device, and heated so that a vaporized raw material did not condense.

A tube of the thermal decomposition reactor was heated at 440°C by electromagnetic induction heating, and the vaporized raw material introduced into the tube was thermally decomposed under the decompression conditions, to obtain N-vinylacetamide.

On the above conditions, a continuous operation was performed for 40 days, but no particular problem occurred. A liquefied raw material collected in a collection pot in 40 days had a weight of about 80 kg (corresponding to about 0.4% of 19,200 kg of raw material fed for 40 days).

### Example 2

After end of Example 1, a continuous operation was continuously performed with the same apparatus and on the same operation conditions as in Example 1.

Furthermore, the continuous operation was performed for 30 days, but no particular problem occurred. A liquefied raw material collected in a collection pot in 30 days had a weight of about 220 kg (corresponding to about 1.5% of 14,400 kg of raw material fed for 30 days).

### Example 3

After end of Example 2, a continuous operation was continuously performed with the same apparatus and on the same operation conditions as in Example 1.

Furthermore, the continuous operation was performed for 32 days, but no particular problem occurred. A liquefied raw material collected in a collection pot in 32 days had a weight of about 340 kg (corresponding to about 2.2% of 15,360 kg of raw material fed for 32 days).

### Example 4

After a continuous operation was performed with the same apparatus and on the same operation conditions as in Example 1 for 10 days, the apparatus was stopped. The operation was restarted again without cleaning an evaporator and a thermal decomposition reactor. The continuous operation was performed as it was for 30 days, but no particular problem occurred. A liquefied raw material collected in a collection pot in a total period of an operation period of 40 days and a stop period had a weight of about 110 kg (corresponding to about 0.6% of 19,200 kg of raw material fed for 40 days).

### Comparative Example 1

When a continuous operation was performed with the same apparatus and on the same operation conditions as in Example 1 except that a raw material collection device was removed from the apparatus of Example 1, turbulence in temperature of a thermal decomposition reactor was confirmed on a seventh day from start of the operation, and the apparatus was out of control and stopped on an eighth day. As a result of opening and inspection of the apparatus, it was found that tar and solid coagulum were generated to block an inlet portion of the thermal decomposition reactor (down to a depth of about 20 cm).

### Industrial Applicability

According to the present invention, N-vinylcarboxylic acid amide obtained in a production method is a useful monomer in production of N-vinylcarboxylic acid amide polymer to be used in a coagulant, a liquid absorbent, a thickener, or the like, and additionally in various industrial applications.

### Reference Signs List

10 raw material feeding device
20A, 20B evaporator
21 collection pot
22a gate valve
22b extraction valve
30A, 30B raw material collection device
31 distribution inhibiting section
32 cylindrical part
33 collecting section
34 discharge piping
35a to 35e gate valve
36, 71 pressure pump
36a gate valve
37a nitrogen valve
40 collection pot
41 extraction valve
50 thermal decomposition reactor
60 cooler
70 reaction solution receiver
100 first piping
110 second piping

## Claims

1. A method for producing N-vinylcarboxylic acid amide, comprising:
a feeding step of feeding N-(1-alkoxyethyl)carboxylic acid amide as a raw material to an evaporator,
an evaporation step of evaporating, by the evaporator, the raw material, to form a vaporized raw material, and
a thermal decomposition step of feeding the vaporized raw material to a thermal decomposition reactor, to thermally decompose the raw material, the method for producing N-vinylcarboxylic acid amide, further comprising:
between the evaporation step and the thermal decomposition step, a collection step of collecting, from the raw material fed to the evaporator, a liquid raw material that is not vaporized and a liquid raw material comprising a part of the vaporized raw material that is liquefied,
the collection step being a step of collecting the liquid raw material by a raw material collection device provided between the evaporator and the thermal decomposition reactor.

2. The method for producing N-vinylcarboxylic acid amide according to claim 1, wherein the evaporation step is performed under reduced pressure.

3. The method for producing N-vinylcarboxylic acid amide according to claim 1 or 2, wherein the thermal decomposition step is performed under reduced pressure.

4. The method for producing N-vinylcarboxylic acid amide according to any one of claims 1 to 3, wherein a downstream end of the evaporator is joined to an upstream end of the raw material collection device.

5. The method for producing N-vinylcarboxylic acid amide according to any one of claims 1 to 3, wherein a downstream end of the evaporator is connected to an upstream end of the raw material collection device via a first piping, and
a downstream end of the first piping is at the lowest position in the first piping.

6. The method for producing N-vinylcarboxylic acid amide according to any one of claims 1 to 5, wherein a downstream end of the raw material collection device is joined to an upstream end of the thermal decomposition reactor.

7. The method for producing N-vinylcarboxylic acid amide according to any one of claims 1 to 5, wherein a downstream end of the raw material collection device is connected to an upstream end of the thermal decomposition reactor via a second piping, and
an upstream end of the second piping is at the lowest position in the second piping.

8. The method for producing N-vinylcarboxylic acid amide according to claim 7, wherein the second piping is connected to a side surface or an upper surface of the raw material collection device.

9. The method for producing N-vinylcarboxylic acid amide according to any one of claims 1 to 8, wherein the evaporator is a falling film evaporator.

10. The method for producing N-vinylcarboxylic acid amide according to any one of claims 1 to 8, wherein the evaporator is a forced film evaporator.

11. The method for producing N-vinylcarboxylic acid amide according to any one of claims 1 to 10, wherein the vaporized raw material is fed through the raw material collection device to the thermal decomposition reactor.

12. The method for producing N-vinylcarboxylic acid amide according to any one of claims 1 to 11, wherein the N-(1-alkoxyethyl)carboxylic acid amide is N-(1-methoxyethyl) acetamide.

13. The method for producing N-vinylcarboxylic acid amide according to any one of claims 1 to 12, wherein the N-vinylcarboxylic acid amide is N-vinylacetamide.

14. The method for producing N-vinylcarboxylic acid amide according to any one of claims 1 to 13, further comprising:
feeding the collected liquid raw material to the evaporator.

15. The method for producing N-vinylcarboxylic acid amide according to any one of claims 1 to 14, wherein the raw material collection device comprises:
a cylindrical part through which the vaporized raw material is distributed,
a collecting section with which flow of the vaporized raw material flowing through the cylindrical part partially or entirely collides, and that collects, from the raw material fed to the evaporator, the liquid raw material that is not vaporized and the liquid raw material comprising a part of the vaporized raw material that is liquefied, and
a discharge piping through which the liquid raw material collected in the collecting section is discharged to outside the cylindrical part.

## Patentansprüche

1. Verfahren zur Herstellung von N-Vinylcarbonsäureamid, umfassend:
einen Beschickungsschritt, bei dem N-(1-Alkoxyethyl)carbonsäureamid als Rohmaterial in einen Verdampfer eingespeist wird,
einen Verdampfungsschritt, bei dem das Rohmaterial durch den Verdampfer verdampft wird, um ein verdampftes Rohmaterial zu bilden, und
einen thermischen Zersetzungsschritt, bei dem das verdampfte Rohmaterial einem thermischen Zersetzungsreaktor zugeführt wird, um das Rohmaterial thermisch zu zersetzen, wobei das Verfahren zur Herstellung von N-Vinylcarbonsäureamid ferner umfasst:
zwischen dem Verdampfungsschritt und dem Schritt der thermischen Zersetzung einen Sammelschritt, bei dem aus dem dem Verdampfer zugeführten Rohmaterial ein flüssiges Rohmaterial, das nicht verdampft ist, und ein flüssiges Rohmaterial, das einen Teil des verdampften Rohmaterials umfasst, der verflüssigt ist, gesammelt wird,
wobei der Sammelschritt ein Schritt des Sammelns des flüssigen Rohmaterials durch eine Rohmaterialsammelvorrichtung, die zwischen dem Verdampfer und dem thermischen Zersetzungsreaktor vorgesehen ist.

2. Verfahren zur Herstellung von N-Vinylcarbonsäureamid nach Anspruch 1, wobei der Verdampfungsschritt unter vermindertem Druck durchgeführt wird.

3. Verfahren zur Herstellung von N-Vinylcarbonsäureamid nach Anspruch 1 oder 2, wobei der thermische Zersetzungsschritt unter vermindertem Druck durchgeführt wird.

4. Verfahren zur Herstellung von N-Vinylcarbonsäureamid nach einem der Ansprüche 1 bis 3, wobei ein stromabwärtiges Ende des Verdampfers mit einem stromaufwärtigen Ende der Rohmaterialsammelvorrichtung verbunden ist.

5. Verfahren zur Herstellung von N-Vinylcarbonsäureamid nach einem der Ansprüche 1 bis 3, wobei ein stromabwärts gelegenes Ende des Verdampfers mit einem stromaufwärts gelegenen Ende der Rohmaterialsammelvorrichtung über eine erste Rohrleitung verbunden ist, und
ein stromabwärts gelegenes Ende der ersten Rohrleitung sich an der niedrigsten Stelle in der ersten Verrohrung befindet.

6. Verfahren zur Herstellung von N-Vinylcarbonsäureamid nach einem Ansprüche 1 bis 5, wobei ein stromabwärtiges Ende der Rohmaterialsammelvorrichtung mit einem stromaufwärtigen Ende des thermischen Zersetzungsreaktors verbunden ist.

7. Verfahren zur Herstellung von N-Vinylcarbonsäureamid nach einem Ansprüche 1 bis 5, wobei ein stromabwärts gelegenes Ende der Rohmaterialsammelvorrichtung über eine zweite Rohrleitung mit einem stromaufwärts gelegenen Ende des thermischen Zersetzungsreaktors verbunden ist, und
ein stromaufwärts gelegenes Ende der zweiten Rohrleitung sich an der tiefsten Stelle der zweiten Rohrleitung befindet.

8. Verfahren zur Herstellung von N-Vinylcarbonsäureamid nach Anspruch 7, wobei die zweite Rohrleitung mit einer Seitenfläche oder einer oberen Fläche der Rohmaterialsammelvorrichtung verbunden ist.

9. Verfahren zur Herstellung von N-Vinylcarbonsäureamid nach einem der Ansprüche 1 bis 8, wobei der Verdampfer ein Fallfilmverdampfer ist.

10. Verfahren zur Herstellung von N-Vinylcarbonsäureamid nach einem der Ansprüche 1 bis 8, wobei der Verdampfer ein Zwangsfilmverdampfer ist.

11. Verfahren zur Herstellung von N-Vinylcarbonsäureamid nach einem der Ansprüche 1 bis 10, wobei das verdampfte Rohmaterial durch die Rohmaterialsammelvorrichtung in den thermischen Zersetzungsreaktor geleitet wird.

12. Verfahren zur Herstellung von N-Vinylcarbonsäureamid einem der Ansprüche 1 bis 11, wobei das N-(1-Alkoxyethyl)carbonsäureamid N-(1-Methoxyethyl)acetamid ist.

13. Verfahren zur Herstellung von N-Vinylcarbonsäureamid nach einem Ansprüche 1 bis 12, wobei das N-Vinylcarbonsäureamid N-Vinylacetamid ist.

14. Verfahren zur Herstellung von N-Vinylcarbonsäureamid nach einem Ansprüche 1 bis 13, das ferner umfasst: Zuführung des gesammelten flüssigen Rohmaterials zum Verdampfer.

15. Verfahren zur Herstellung von N-Vinylcarboxylsäureamid nach einem der Ansprüche 1 bis 14, wobei die Rohmaterialsammelvorrichtung folgendes umfasst:
einen zylindrischen Teil, durch den das verdampfte Rohmaterial verteilt wird,
einen Sammelabschnitt, mit dem der Strom des durch den zylindrischen Teil fließenden verdampften Rohmaterials teilweise oder vollständig zusammenstößt und der aus dem dem Verdampfer zugeführten Rohmaterial das flüssige Rohmaterial, das nicht verdampft ist, und das flüssige Rohmaterial, das einen Teil des verdampften Rohmaterials enthält, der verflüssigt ist, sammelt, und
eine Abflussleitung, durch die das im Sammelabschnitt gesammelte flüssige Rohmaterial nach außerhalb des zylindrischen Teils abgeleitet wird.

## Revendications

1. Procédé destiné à produire un amide d'acide N-vinylcarboxylique, comprenant :
une étape d'alimentation destinée à alimenter un amide d'acide N-(1-alcoxyéthyl)carboxylique en tant que matière première dans un évaporateur,
une étape d'évaporation destinée à évaporer, par l'évaporateur, la matière première, pour former une matière première vaporisée, et
une étape de décomposition thermique destinée à alimenter la matière première vaporisée dans un réacteur de décomposition thermique, pour décomposer thermiquement la matière première, le procédé de production d'amide d'acide N-vinylcarboxylique comprenant en outre :
entre l'étape d'évaporation et l'étape de décomposition thermique, une étape de récupération destinée à récupérer, à partir de la matière première alimentée dans l'évaporateur, une matière première liquide qui n'est pas vaporisée et une matière première liquide comprenant une partie de la matière première vaporisée qui est liquéfiée,
l'étape de récupération étant une étape destinée à récupérer la matière première liquide par un dispositif de récupération de matière première prévu entre l'évaporateur et le réacteur de décomposition thermique.

2. Procédé de production d'amide d'acide N-vinylcarboxylique selon la revendication 1, dans lequel l'étape d'évaporation est réalisée sous pression réduite.

3. Procédé de production d'amide d'acide N-vinylcarboxylique selon la revendication 1 ou 2, dans lequel l'étape de décomposition thermique est réalisée sous pression réduite.

4. Procédé de production d'amide d'acide N-vinylcarboxylique selon l'une quelconque des revendications 1 à 3, dans lequel une extrémité en aval de l'évaporateur est reliée à une extrémité en amont du dispositif de récupération de matière première.

5. Procédé de production d'amide d'acide N-vinylcarboxylique selon l'une quelconque des revendications 1 à 3, dans lequel une extrémité en aval de l'évaporateur est reliée à une extrémité en amont du dispositif de récupération de matière première par le biais d'une première tuyauterie, et
une extrémité en aval de la première tuyauterie est à la position la plus basse dans la première tuyauterie.

6. Procédé de production d'amide d'acide N-vinylcarboxylique selon l'une quelconque des revendications 1 à 5, dans lequel une extrémité en aval du dispositif de récupération de matière première est reliée à une extrémité en amont du réacteur de décomposition thermique.

7. Procédé de production d'amide d'acide N-vinylcarboxylique selon l'une quelconque des revendications 1 à 5, dans lequel une extrémité en aval du dispositif de récupération de matière première est reliée à une extrémité en amont du réacteur de décomposition thermique par le biais d'une seconde tuyauterie, et une extrémité en amont de la seconde tuyauterie est à la position la plus basse dans la seconde tuyauterie.

8. Procédé de production d'amide d'acide N-vinylcarboxylique selon la revendication 7, dans lequel la seconde tuyauterie est reliée à une surface latérale ou une surface supérieure du dispositif de récupération de matière première.

9. Procédé de production d'amide d'acide N-vinylcarboxylique selon l'une quelconque des revendications 1 à 8, dans lequel l'évaporateur est un évaporateur à film tombant.

10. Procédé de production d'amide d'acide N-vinylcarboxylique selon l'une quelconque des revendications 1 à 8, dans lequel l'évaporateur est un évaporateur à film forcé.

11. Procédé de production d'amide d'acide N-vinylcarboxylique selon l'une quelconque des revendications 1 à 10, dans lequel la matière première vaporisée est alimentée à travers le dispositif de récupération de matière première dans le réacteur de décomposition thermique.

12. Procédé de production d'amide d'acide N-vinylcarboxylique selon l'une quelconque des revendications 1 à 11, dans lequel l'amide d'acide N-(1-alcoxyéthyl)carboxylique est le N-(1-méthoxyéthyl)acétamide.

13. Procédé de production d'amide d'acide N-vinylcarboxylique selon l'une quelconque des revendications 1 à 12, dans lequel l'amide d'acide N-vinylcarboxylique est le N-vinylacétamide.

14. Procédé de production d'amide d'acide N-vinylcarboxylique selon l'une quelconque des revendications 1 à 13, comprenant en outre :
alimenter la matière première liquide récupérée dans l'évaporateur.

15. Procédé de production d'amide d'acide N-vinylcarboxylique selon l'une quelconque des revendications 1 à 14, dans lequel le dispositif de récupération de matière première comprend :
une partie cylindrique à travers laquelle la matière première vaporisée est distribuée,
une section de récupération, avec laquelle l'écoulement de la matière première vaporisée s'écoulant à travers la partie cylindrique entre en collision partiellement ou entièrement, et
qui récupère, à partir de la matière première alimentée dans l'évaporateur, la matière première liquide qui n'est pas vaporisée et la matière première liquide comprenant une partie de la matière première vaporisée qui est liquéfiée, et
une tuyauterie d'évacuation à travers laquelle la matière première liquide récupérée dans la section de récupération est évacuée vers l'extérieur de la partie cylindrique.
